# EUROPEAN PATENT SPECIFICATION

(11) **EP 1 931 445 B1**
(45) Date of publication and mention of the grant of the patent: **01.08.2018**
(21) Application number: 06791737.7
(22) Date of filing: 30.08.2006
(51) Int. Cl.: B01D 39/16

(54) **CYCLIC OLEFIN COPOLYMER AS A LEUKOREDUCTION MATERIAL**
CYCLOOLEFINCOPOLYMER ALS MATERIAL ZUR VERMINDERUNG DES GEHALTS AN LEUKOZYTEN
COPOLYMERE D'OLEFINE CYCLIQUE UTILISE EN TANT QUE MATERIAU DE LEUCOREDUCTION

(30) Priority: 31.08.2005 US 713527 P; 10.04.2006 US 744571 P
(43) Date of publication of application: 18.06.2008
(73) Proprietor: Gambro Lundia AB, 226 43 Lund (SE)
(72) Inventor: HAGG, Sylvia, 72458 Albstadt (DE); DEPPISCH, Reinhold, 72379 Hechingen (DE); WITTNER, Bernd, 72379 Hechingen (DE)
(74) Representative: Hornung, Veronika Margot
(86) International application number: PCT/EP2006/008487
(87) International publication number: WO 2007/025738

(56) References cited:
- EP-A1- 0 554 460
- EP-A2- 0 188 104
- WO-A-98/56836
- DATABASE WPI Week 199507 Derwent Publications Ltd., London, GB; AN 1995-049477 XP002427751 & JP 06 330445 A (IDEMITSU KOSAN CO LTD) 29 November 1994 (1994-11-29)

## Description

### FIELD OF THE INVENTION

The present invention relates to the use of a media for leukoreduction of blood or blood components, a leukoreduction filter and a method of conditioning the media.

### BACKGROUND

It has been the practice for 50 years or more to transfuse whole blood, and more recently blood components, from one or more donors to other patients. With the passage of time and accumulation of research and clinical data, transfusion practices have improved greatly. One aspect of current practice is that whole blood is rarely administered; rather, patients needing red blood cells are given packed red blood cells, and patients needing platelets are given platelet concentrates and plasma. These components are usually separated from whole blood by centrifugation.

In addition to the components listed above (platelets, plasma and red blood cells) whole blood also contains white blood cells (known collectively as leukocytes.) White blood cells provide protection against bacterial and viral infections. However, it has been estimated that as much as ninety percent of adverse reactions from blood transfusions are caused by these donor leukocytes.

In current practice, it is desirable to reduce the leukocyte concentration of blood components to as low a level as possible. While there is no firm criterion, it is generally accepted that many of the undesirable effects of transfusion would be adequately reduced if the leukocyte content were reduced by a factor of about 100 or more prior to administration of the blood component to the patient. This approximates reducing the total content of leukocytes in a single unit of packed red blood cells (PRC) (the quantity of PRC obtained from a single blood donation) to less than 0.1 x 10⁹.

One method of reducing leukocytes is through the use of a leukoreduction filter. In order to make an efficient white blood cell or leukoreduction filter, several parameters of the filter material or media are considered. Adhesion of the cells to the filter media or material, and the mechanical retention of the cells by the media or material are two parameters that help determine efficiency of leukocyte capture.

Cell adhesion can be achieved by techniques relating to the physical characteristics of the surfaces of the filter media such as surface energy and charge. Surface charge can be modified through selection of fiber material for the filter media or material and through surface modification of the selected material.

Other characteristics of the surface of the filter media may also be modified in order to achieve better cell adhesion and thus better filtration. One such characteristic which may be modified in order to increase filtration efficiency is the "wettability" of the media or material. When a liquid is brought into contact with the upstream surface of a porous medium and a small pressure differential is applied, flow into and through the porous medium may or may not occur, depending upon the wettability of the fiber. The more "wettable" a material is, the easier liquid will penetrate through the fibers. A condition in which no flow occurs is that in which the liquid does not wet the porous filter material. Such lack of fluid flow through a filter significantly decreases the efficiency of the filtration medium, increases filter clogging and significantly increases filtration time.

The wettability of the media can be modified by a number of methods, forexample by chemical reaction including dry or wet oxidation, by coating the fiber surface by depositing a polymer thereon, and by surface grafting reactions which are activated by exposure to an energy source such as heat, a Van der Graff generator, ultraviolet light or to various other forms of radiation, among which gamma radiation is most often used.

Mechanical retention is achieved by the pore size of the material or media. The pore size is achieved through a combination of fiber diameter and fiber compression. Pore size is typically defined as the distance between two adjacent fibers.

Selection of fiber diameter is also an important parameter. As mentioned above, adsorption of leukocytes on fiber surfaces is widely accepted as the mechanism of leukocyte removal. Since the surface area of a given weight of fibers is inversely proportional to the diameter of the fibers, and adsorption to the fiber surfaces is a significant mechanism for leukocyte depletion, it is expected that finer fibers will have higher retention capacity and that the fiber quantity, as measured by weight of fibers necessary to achieve a desired efficiency, will be less if the fibers used are smaller in diameter.

Mechanical retention (or sieving) of cells by the media as noted above, can be further increased by using fiber diameters which are as small as possible. Melt blowing, in which molten resin is attenuated into fibers by a high velocity stream of gas and collected as a non-woven web, came into production in the 1960's and 1970's and has been gradually extended over the years with respect to the lower limit of fiber diameter with which webs could be made. Within recent years, webs with fiber diameters less than two micrometers have been made.

Some resins are better adapted to melt blowing of fine fibers than others. Resins which work well include polypropylene, polymethylpentene, Nylon 6, polyester PET (polyethylene terephthalate), and polyester PBT (polybutylene terephthalate). Polyester PBT has been the principal resin used for the development of leukocyte filters, because it lends itself to radiation grafting and to subsequent conversion into preformed elements of controlled pore size by hot pressing.

One disadvantage of the currently used resins is that to achieve acceptable leukoreduction of blood products, polyester material such as PBT requires additional treatment of the material before it can be used. Treatments to achieve desired surface charge (also called zeta potential) and desired wettability require additional processing steps before the media is assembled into a filter.

It is thus desirable to find a resin material, which has chemical characteristics that would enable the material to be used as leukoreduction media, and which would avoid the extra processing steps described above. It is to such a new material that the present invention is directed.

### SUMMARY OF THE INVENTION

An embodiment of this invention is directed toward a leukoreduction filter for selectively removing leukocytes from blood or a blood product. The filter is made of an inert hydrophobic material having a wettability of 44 dynes/cm melt-blown into fibers.

The inert hydrophobic material is a non-woven melt-blown web of cyclic olefin copolymer fibers, wherein the fibers are preconditioned with a material to increase wettability of the fibers.

Also claimed is a method of conditioning the melt-blown COC media with Tween® 20. The method includes flowing a conditioning solution containing the desired amount of Tween® 20 through the media to condition the media and rinsing the conditioned media with saline for an amount of time sufficient to remove substantially all residual Tween® 20.

### BRIEF DESCRIPTION OF THE DRAWINGS

FIG. 1 is a graph showing the range of cycloolefin copolymer fiber diameters produced during the melt-blown process.
FIG. 2 is a scanning electron micrograph of the fibers of the present invention.
FIG. 3. is a graph showing a pore size distribution analysis of the melt-blown cycloolefin copolymer (COC) material.
FIG. 4a and 4b show two processes for treating the membrane of this invention with a conditioning solution.
FIG. 5 is a graph comparing thrombin-anti-thrombin III activity (TAT) of untreated COC and COC treated with albumin.
FIG. 6 is a graph comparing terminal complement complex activity of untreated COC and COC treated with albumin.
FIG. 7 is a graph comparing the amount of kallikrein generated by untreated COC and COC treated with albumin.
FIG. 8 is a graph showing that COC has no negative effect on cell growth.
FIG. 9a is a graph of cell counts v. perfusion time through COC pre-treated with Tween® 20.
FIG. 9b is a graph of cell counts v. perfusion time through untreated COC.
FIG. 10 is a graph of TAT generation after a 40 minute exposure to untreated COC and COC pre-treated with SMA.
FIG. 11 is a graph comparing cell counts after perfusion through untreated and treated COC.
FIG. 12 is a graph comparing the residual Tween® 20 left in the COC after a conditioning procedure.
FIG. 13 is a cross sectional view of a leukoreduction filter which may be made with COC media.

### DETAILED DESCRIPTION

The present invention is directed toward the use of media/material in a leukoreduction filter which is made from a cyclic olefin copolymer resin, hereinafter referred to as COC. The terms media and material are used interchangeably. COC is an amorphous, transparent copolymer based on cycloolefins and linear olefins. Cyclic olefin copolymers (COC) are copolymers of ethylene and a ring-structured olefin, typically derived from dicyclopentadiene.

COC resin may be suitable for use in leukoreduction media for several reasons. The material is inert, which enables it to be used without additional modifications of the material, such as those described in the background above. If modification of the material is desired however, the inert characteristics of the COC aids in such modification.

The chemical composition of COC enables it to be melt-blown to form a web or non-woven mat of fiber material or media.

COC resin is sold by Topas Advanced Polymers (Frankfurt, DE.) The table below lists some of the physical properties of COC resin.

| | |
|---|---|
| Density | 1.02 g/cm² |
| Volume flow index MVR (at 260 °C) | 48 cm³/10 min |
| Melting range | 240-300 °C |
| Melt density (at 240 °C) | 0.91 g/cm³ |

COC is an inert compound. This means that no molecular charges from the material itself should interfere with the ability of leukocytes to bind to the material. It has neither a positive nor negative zeta potential.

Because COC is a low density, amorphous material, the resin is easy to form into material or media of non-woven, melt blown webs. U.S. Patent 6,927,184 describes a process for melt blowing COC into webs.

The melt-blowing process results in fibers with diameters of between 0.1 to 20 µm. In the melt-blowing process, the fiber-forming cycloolefin polymer resin is melted in an extruder and brought to the appropriate temperature and extruded through an array of a multiplicity of extrusion orifices into a high velocity hot air stream. The rapidly moving hot air attenuates the melt streams, creating fibers in the microdenier region. The die arrangement is generally a linear array of capillaries having a small diameter. Typical capillary hole diameters of the die range from 0.25 to 0.5 millimeters. The air stream impinges on the melt filaments extruded by the holes from both sides. The air stream can have a temperature of approximately 100 to 300 °C. The ambient air drawn into the hot air stream cools the hot gas and solidifies the fibers. The melt-blown fibers are collected on a receiver in the path of the fiber stream to form a non-woven mat or web.

Parameters of the melt-blowing process may be varied to achieve melt-blown webs suitable for different applications. For example, if it is desired to use the COC resin as media in leukoreduction applications, the characteristics of the web may be modified during the melt-blowing and manufacturing process. The table below lists some preferred parameters of the melt-lowing process that may be used to produce fiber webs having characteristics which lend themselves toward leukoreduction applications.

| | |
|---|---|
| Process hot air stream (m³/h) | > 400-1000 |
| Through-put polymer mass/hole (g/min) | 0.35-.02 |
| Capillary diameter of the spinning die (mm) | 0.3-.02 |
| Capillary tip hole /inch (number) | < 12 hole/ <30 inch |
| Melt temperature of resin | 300-320 °C |

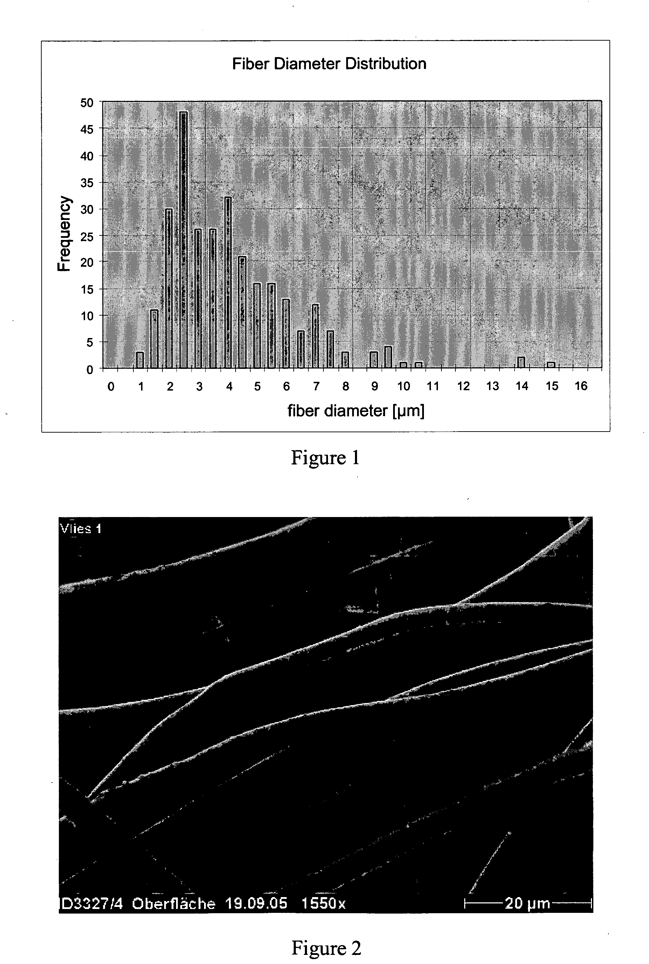

COC has a low melt flow index characterized by the molecular structure of the COC resin. The melt flow index (cm³/10 min) may be between around 200 to 340°C. At a melt temperature of around 300-320 °C, the fibers which are generated during the melt-blowing process have a range of fiber diameters between about 1 to about 8 µm. This range is shown in Fig. 1.

The ranges of fiber diameters produced by the melt-blown process is shown in the scanning electron microscope picture of Fig. 2.

The desired porosity of the COC media is around 80%. This means that 80 % of the media is air, 20% is solid material (fibers).

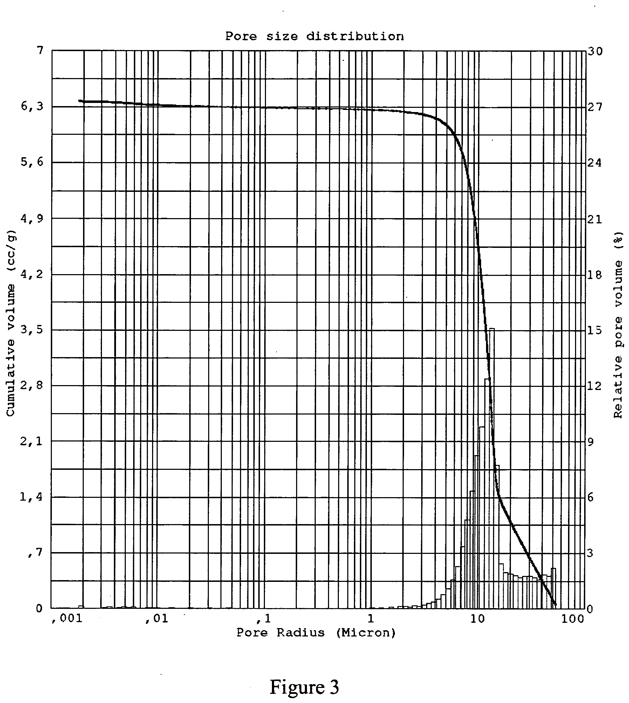

Fig. 3 shows a pore size distribution analysis of the melt-blown non-woven COC material. This shows the average pore radius in microns and the associated percentage of this pore radius in the non-woven material (relative pore volume %). This is determined using a standard method called "mercury porosimetry". As can be seen, the pores are centered around the range of 2-20µm.

The wettability for untreated COC media was determined to be 44 dynes/cm. This is shown in Table 1 below. Wettability is determined by measuring the contact angles of the COC fibers. Contact angle measurement is a standard method to detect surface energy of a solid. When a liquid is dropped onto a surface there are surface energies which occur between the liquid and the surface. This contact angle a can be measured. With this data and a calculation method (Owens Wendt Rabbel Kaelble calculation method) the surface energy of the material (SE) can be calculated. The total surface energy (SE total) consists of dispersive + polar components. mN/m is equal to dynes/cm.

**Table 1**

| | Value (mN/m) |
|---|---|
| SE (total) | 44.89 |
| Dispersive point | 44.23 |
| Polar point | 0.66 |

Another advantage of COC material is that it can be primed by wet steam sterilization. When using wet steam as the sterilant, the steam wets the material, subsequently priming the material.

Wetting the COC material could also be done immediately before use by priming the material with cell storage solution, or the cell storage solution could be pulled through the material using negative pressure. The wetted material may also be stored before use in a cell storage or other solution.

Unlike other commonly-used polymeric materials, COC resin is inert. Because of its inert character, no modification of the melt-blown fibers is necessary to increase biocompatibility. Therefore, no plasma treatment or other type of surface grafting is needed. However, if such treatment is desired, the inertness of the COC resin makes surface modification easier than it would be for less-inert resins.

Standard biocompatibility testing assays were used to measure the biocompatibility of the untreated COC media. Assays to measure TAT (Thrombin Anti-Thrombin III), TCC (Terminal Complement Complex), Copha (Contact phase activation) and ICG (Inhibition of Cells Growth) were done to determine what effect, if any, the media had on cell growth and cell viability.

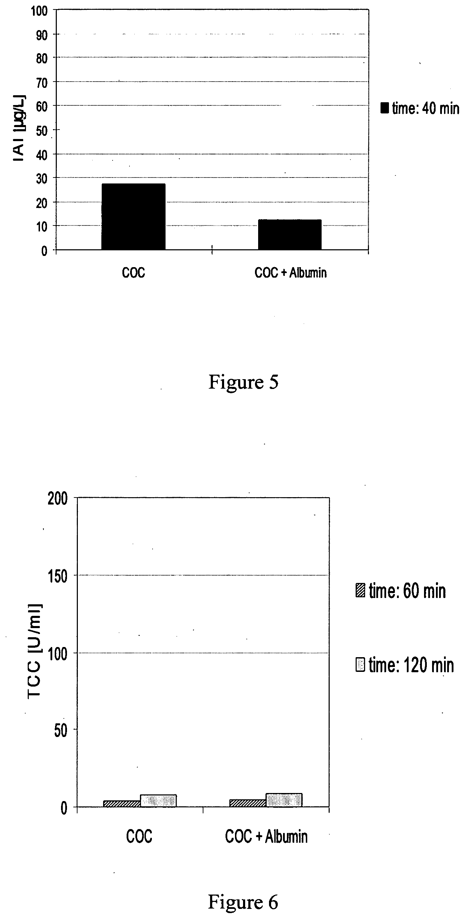

TAT was measured using a standard kit available from Haemochrome (Behring Diagnostics GmbH, D-35041 Marburg, DE). TAT is a measure of the effect something has -in this case the material- on thrombin activation (clotting cascade, extrinsic pathway), which causes blood coagulation. To be considered biocompatible with blood, the material should not cause blood coagulation and should generate a low TAT signal. As shown in Figure 5, both COC material alone and COC material with albumin (albumin inhibits thrombin activation) generates low TAT signal. In comparison, polystyrene material, which is not considered to be biocompatible, generates a high TAT signal.

TCC is a standard test to measure activation of the complement cascade. If COC material is biocompatible with blood, the TCC test should produce low complement activity. Results for untreated COC material and COC material treated with albumin (albumin inhibits high complement activity) are shown in Fig. 6.

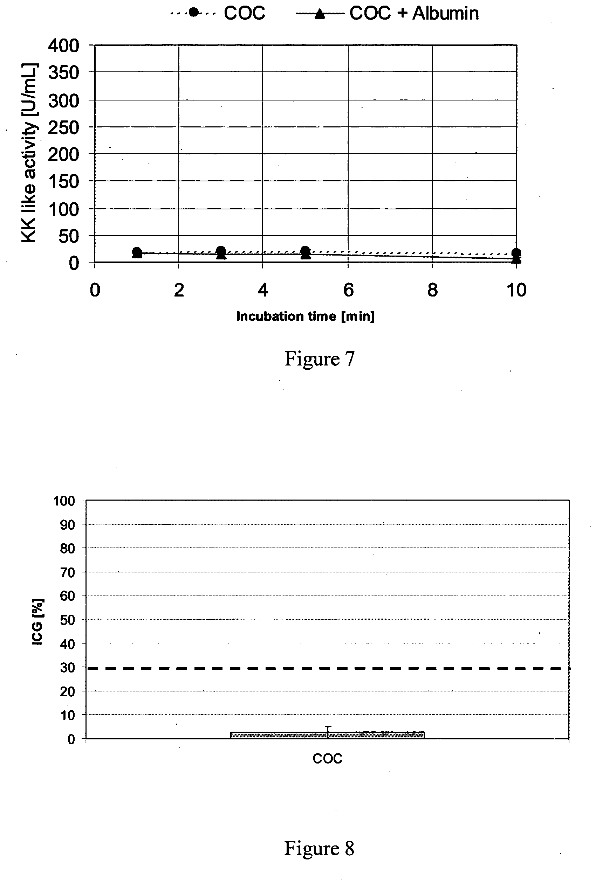

Copha is another standard test used to measure activation of the intrinsic pathway of the clotting cascade. Copha measures the amount of kallikrein (kk), which is generated upon contact with the surface of the material- in this case COC. Activation is measured in kallikrein-like activity. If COC is biocompatible, the Copha test should also produce low clotting factor activity. Results are shown in Figure 7. As expected, COC alone and COC with albumin (albumin has low clotting factor activity) produces low kk-like activity (COC does not activate the clotting cascade).

To test the effect of COC media on cell growth, a standard neutral red uptake assay was done. Human fibroblast cell line L929 is exposed to the eluate of COC media. The eluate and cells are incubated for 72 hours at 37°C. The cells are stained with neutral red stain. The nuclei of live cells stain red, dead cells do not stain. The graph in Fig. 8 shows that cells grown in eluate from cells exposed to COC material do not induce any negative effect on cell growth. Materials above 30% are classified as toxic.

In the blood filter art, it is common practice to coat the surface of the leukoreduction material which will be exposed to blood with additives to increase surface characteristics such as bio compatibility or wettability. Other commonly used leukoreduction filter material such as polyester has a chemical structure which does not allow additives to be directly mixed into the material before the melt-blowing process. Typically, such additives are added directly to the surface of the fibers after the melt-blowing process. Because of the inert nature of the COC resin, if it is desirable to add additional additives to the material to further increase surface characteristics, it is believed that additional additives may be added to the COC resin before or during the melt-blowing process. The ability to add additives to the resin before or during the melt-blowing process provides an advantage over the prior art because it saves a step. However, additives to increase biocompatibility can also be applied directly to the surface of the fibers.

One additive which may be used to further increase the biocompatibility of the COC material is polysiloxane co-polymer, or SMA.

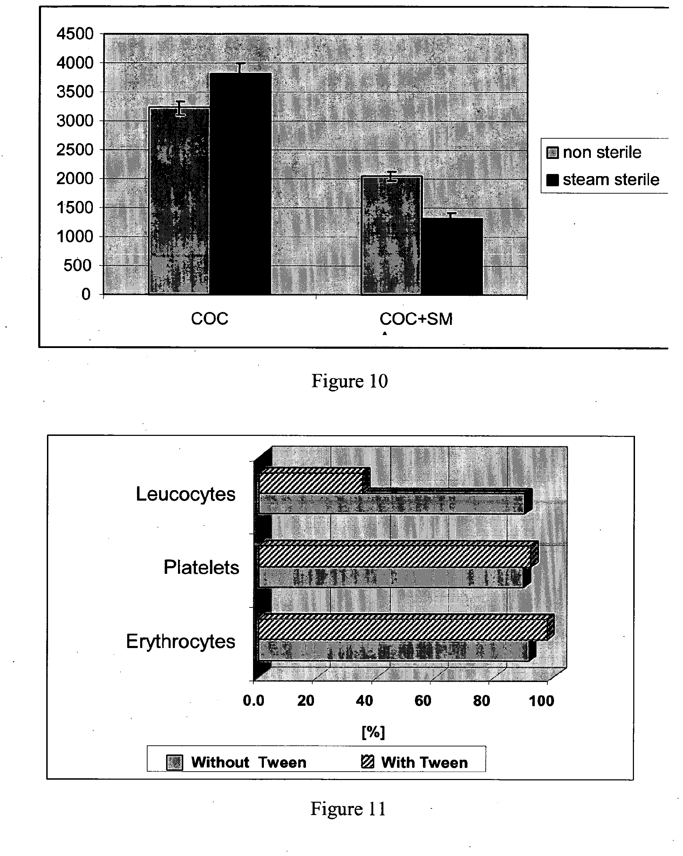

Fig. 10 shows TAT generation after a 40-minute exposure to SMA modified COC media and untreated COC media, with and without steam sterilization. As can be seen, SMA appears to increase the biocompatibility of the COC material, as less signal is generated by SMA treated material.

Other additives which may be used to increase the wettability of the COC material are polysorbate compounds. Tween® 20 or Polysorbate 20 (polyoxyethylene-sorbitane monolaurate) is one such additive also known from EP 0 188 104. In combination with the COC material, Tween® 20 has several functions which may enable better therapeutic functionality of the material as a leukoreduction filter. As noted above with respect to biocompatibility, the inertness of the COC itself also aids in ease of use of conditioning materials.

Conditioning the material with Tween® 20 results in increased wettability. The hydrophobic material which has an original wettability of 44 dyn/cm increases to 72 dyn/cm (the surface tension of water) after treatment with Tween® 20.

Increasing fiber wettability also modulates the separation characteristics of the media by defining the distribution space for different cell sizes, which helps to determine specific or preferential removal or capture of particular cells. For example, leukocytes may be preferentially removed by the filter, while allowing red blood cells and platelets to flow through the media. Alternatively, platelets and leukocytes may be preferentially removed by the filter while red blood cells flow through.

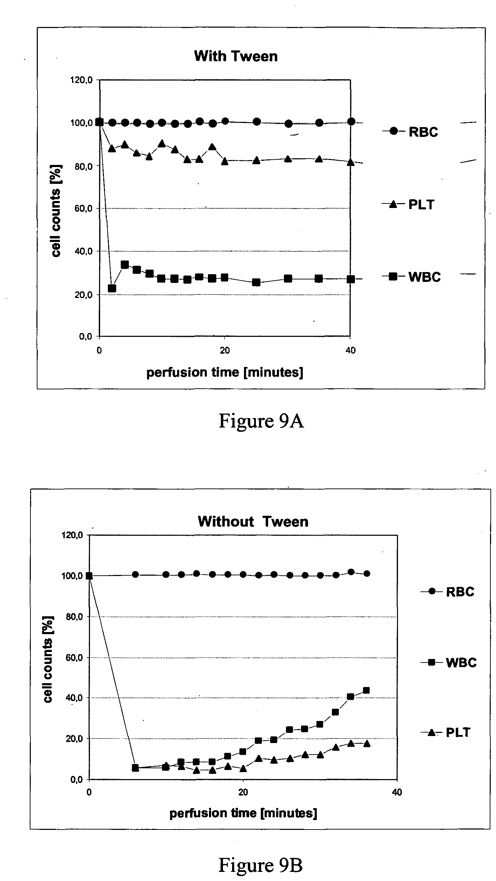

Figs. 9a and 9b are graphs of cell counts of a whole blood perfusion experiment with and without pre-conditioning the media with Tween® 20. As shown in Fig. 9a, preconditioning the media with 0.2% Tween® 20 appears to improve leukocyte (WBC) removal up to about 80% with very little platelet (PLT) and red blood cell RBC) loss. The non-conditioned media (Fig. 9b) appears to trap not only leukocytes but also platelets.

COC media conditioned with Tween® 20 also appears to prevent erythrocyte loss. In these experiments, human whole blood was pumped through filters made of COC. One filter was conditioned with (0.2%) Tween® 20 solution at 37 °C immediately before the experiment began, the other was not conditioned. After conditioning the filter, both filters were rinsed with 2 L saline. Whole blood was split into two flasks each containing 250 mL. The blood was circulated through the filters at a flow rate of 50 mL/min at 37 °C. Samples were taken from the blood in and blood out sides of the filter. The samples were analyzed using a Sysmex 10C-21 cell counter. The results are shown in Table 2 below. As can be seen, conditioning COC with Tween® 20 enables better cell separation than non-conditioned COC.

**Table2**

| Cell Fraction | +Tween Cell counts [10³/µL] | | -Tween Cell counts [10³/µL] | |
|---|---|---|---|---|
| | Blood ᵢₙ | Blood ₒᵤₜ | Blood ᵢₙ | Blood ₒᵤₜ |
| leukocytes | 5.4 | 1.9 | 7.1 | 6.4 |
| erythrocytes | 4.7 | 4.6 | 4.7 | 4.3 |
| thrombocytes | 219 | 202 | 155 | 139 |

A comparison of cell counts after filter contact in accordance with the test described above with reference to Table 2, are shown in Fig. 11. As corroborated by Table 2, substantially all erythrocytes and platelets flowed through the conditioned filter. The majority of leucocytes were trapped by the conditioned COC material, as opposed to the non-conditioned media, which allowed the majority of leucocytes to flow through.

It therefore follows from these experiments that changing the concentration of Tween® 20 may change the type of cells which are captured by the material. For example, reducing the concentration of Tween® 20 may increase the capture of platelets by the material.

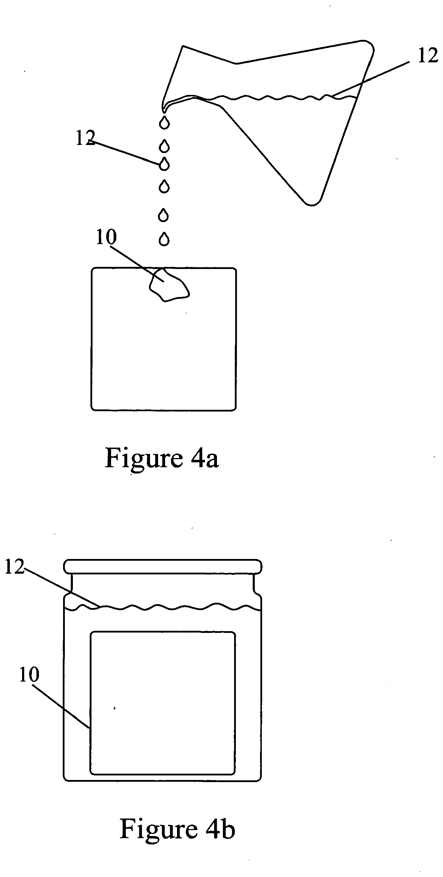

Conditioning the COC material may be accomplished in several ways. The material **10** may be conditioned before use by exposing the media to a conditioning solution by rinsing/washing/flowing the solution **12** containing 50 mL aqueous non-buffered solution and 2 mg/mL (0.2%) Tween® 20. This process is shown in FIG. 4a. The material **10** may also be conditioned by soaking or suspending the material **10** in a 0.2% Tween® 20 solution **12** for a period of time. This process is shown in FIG. 4b. The Tween® 20 containing solution may be applied immediately before use of the filter followed by subsequent rinsing of the device by a saline solution, or a Tween® 20 solution may be added during the manufacturing process before the filter is sterilized. The conditioning solution may also be added before the sterilization process.

The residual Tween® 20 left in the COC material after conditioning was studied. Four cell filters made of COC material were conditioned either in a static (soaking or suspending) or dynamic (flowing) manner with Tween® 20 solution. Tween® 20 was prepared in water. Filter 1 was conditioned with Tween® 20 at a concentration of 0.1% (v/v). Filters 2, 3 and 4 were conditioned with Tween® 20 at a concentration of 0.2%. Filters 1 and 2 were filled and circulated at 50 mL/min with 500 mL conditioning solution (dynamic process.) Filters 3 and 4 were filled with 50 mL conditioning solution and incubated (static process.) Filter 3 was incubated for 15 minutes and filter 4 was incubated for 60 minutes. All filters were rinsed single pass with 1 L distilled water at 37 °C. At 0, 2, 3, 4, 6, 8, 10, 15, 20, 30, 35 and 40 minute time intervals, samples were taken from the filters. The surface tension in dyn/cm of the samples was determined.

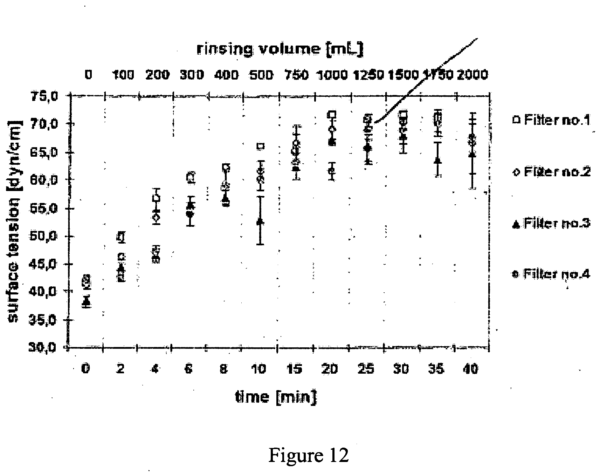

As shown in Fig. 12, the results show a surface tension from 40 dyns/cm at the beginning of testing increasing to 70 +/- 2 dyns/cm during the rinsing procedure. This means that after rinsing with 1 L of distilled water there is hardly any residual Tween® 20 left in the material. 72 dyns/cm is the surface tension of water. As can be seen, there is no difference in static vs. dynamic conditioning of the COC material.

An ICG test was performed using the protocol as described above, to study the toxicity levels in the samples. As seen in Table 3 below, toxicity levels for each filter are shown for each measured time period. As seen, there is a generally decreasing trend of cell toxicity from T= 0 to T= 40 in all samples. Time = 0 shows the Tween® 20 conditioning solution at the start of the experiment. After 4 minutes or rinsing with 200 mL distilled water, the toxicity level is below 30. After 8 minutes or rinsing with 400 mL water, the toxicity level is below 20. Whether the filters were conditioned in a static or dynamic manner has no effect on the ability of Tween® 20 to be rinsed out of the COC material.

**Table 3**

| Filter No. | Time (T) (min) | | | | | |
|---|---|---|---|---|---|---|
| | 0 | 4 | 8 | 25 | 35 | 40 |
| 1 | 94 | 0 | 8 | 17 | 2 | --- |
| 2 | 100 | 28 | --- | --- | 2 | 18 |
| 3 | 100 | --- | --- | 19 | 0 | 0 |
| 4 | 100 | 29 | 0 | --- | --- | --- |
| ICG | > 30% | < 30% | < 30% | < 30% | < 30% | < 30% |

According to the FDA-CDER (FDA Center for Drug Evaluation and Research) the recommendation for intravenous infusion of Tween® 20 for maximum potency is between 0.4-2.4%. In the above experiments, the concentration of Tween® 20 conditioning solution was 2-4 fold below the lowest FDA-CDER recommendations. After a minimal rinsing with 1 L of water, the toxicity of each sample (Filters 1-4) is below 30 %. The COC leukoreduction filter conditioned with Tween® 20 is certainly well below toxicity standards for human use.

The conditioning solution and process as described above may also be used to enhance the functional profile of hydrophobic materials other than COC.

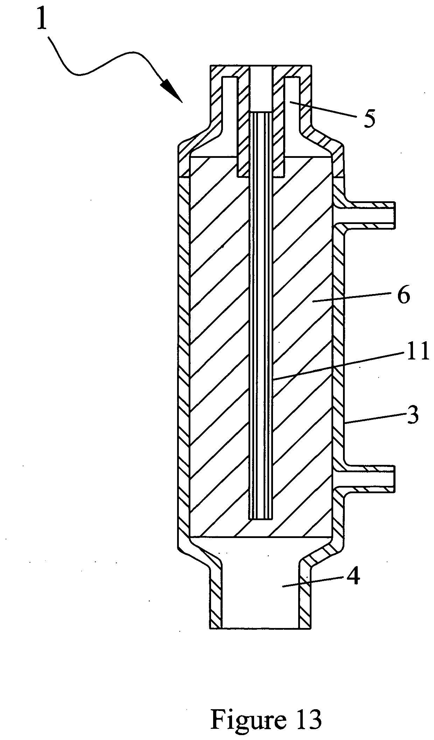

The treated and/or untreated COC material may be made into a leukoreduction filter as is known in the art. One such filter is shown in cross section in FIG. 13. The filter **1** of this invention may consist of at least a housing **3** and a first end **4** and a second end **5.** COC media **6** is contained within the housing **3.** A fluid flow device **11** directs fluid which may be blood or blood components to be leukoreduced through the filter media **3** and out of the housing **3** through end **5.**

The filter may also use a combination of filtration materials, for example, a module may be constructed that utilizes a combination of both treated and untreated COC.

Blood to be leukoreduced may be pumped through the filter, or may flow through the filter using gravity.

Another subject of the invention is the use of an inert hydrophobic media comprising a non-woven melt-blown web of cyclic olefin copolymer fibers wherein the fibers are conditioned with a solution containing Tween® 20 in an amount of between 0.01 to 0.5% and in an amount non-toxic to humans for selectively removing leukocytes from blood or a blood product. In one embodiment, the media has a wettability value of 72 dynes/cm. In one embodiment, the fibers have a diameter of from 1 to 8 µm. In one embodiment, the porosity of the media is between 75-95%, for instance, between 85-90%. In one embodiment, the media comprise pores in the melt blown web and the majority of the pores are 2-30 µm, e.g., 5-15 µm. In one embodiment, the fibers are compressed and mechanically processed by a factor of between 1-10. In one embodiment, the media is wet steam sterilized. In one embodiment, the media is conditioned before use. In a further embodiment, the media is conditioned with a fluid before sterilization.

The invention also provides a leukoreduction filter comprising a media comprising a non-woven melt-blown web of fibers of cyclic olefin copolymer conditioned with a solution containing Tween® 20 in an amount between 0.01 to 0.5%.

Another subject of the invention is a method of conditioning a media comprising a non-woven melt-blown web of cyclic oo1efin copolymer fibers with Tween® 20. The method comprises flowing a conditioning solution through the media or soaking the media in a conditioning solution. The conditioning solution contains 0.01 to 0.5% Tween® 20. The conditioned media is rinsed with saline or water for an amount of time sufficient to remove substantially all residual Tween® 20. In one embodiment, the method further comprises sterilizing the media; either before or after the flowing step.

## Claims

1. Use of an inert hydrophobic media comprising a non-woven melt-blown web of cyclic olefin copolymer fibers wherein the fibers are conditioned with a solution containing Tween® 20 in an amount between 0.01 to 0.5% and in an amount non-toxic to humans for selectively removing leukocytes from blood or a blood product.

2. Use according to claim 1 wherein the media has a wettability value of 72 dynes/cm.

3. Use according to claim 1 wherein the fibers have a diameter of from 1 to 8 µm.

4. Use according to claim 1 wherein the porosity of the media is between 75-95%.

5. Use according to claim 4 wherein the porosity is between 85-90%.

6. Use according to claim 1 wherein the media comprise pores in the melt blown web and wherein the majority of the pores are 2-30 µm.

7. Use according to claim 6 wherein the majority of the pores are 5-15 µm.

8. Use according to claim 1 wherein the fibers are compressed and mechanically processed by a factor of between 1-10.

9. Use according to claim 1 wherein the media is wet steam sterilized.

10. Use according to claim 1 wherein the media is conditioned before use.

11. Use according to claim 9 wherein the media is conditioned before sterilization.

12. A leukoreduction filter comprising a media comprising a non-woven melt-blown web of fibers of cyclic olefin copolymer conditioned with a solution containing Tween® 20 in an amount between 0.01 to 0.5%.

13. A method of conditioning a media comprising a non-woven melt-blown web of cyclic oo1efin copolymer fibers with Tween® 20 comprising the steps of:
flowing a conditioning solution through the media or soaking the media in a conditioning solution; the conditioning solution containing 0.01 to 0.5% Tween® 20; and
rinsing the conditioned media with saline or water for an amount of time sufficient to remove substantially all residual Tween® 20.

14. The method of claim 13 further comprising sterilizing the media; wherein the flowing step occurs before the sterilizing step.

15. The method of claim 13 further comprising sterilizing the media; wherein the flowing step occurs after the sterilizing step.

## Patentansprüche

1. Verwendung eines inerten hydrophoben Mediums, das ein schmelzgeblasenes Vlies aus Cycloolefincopolymerfasern umfasst, worin die Fasern konditioniert sind mit einer Lösung, die Tween® 20 in einer Menge zwischen 0,01 und 0,5% und in einer für Menschen nicht toxischen Menge enthält, zur selektiven Entfernung von Leukozyten aus Blut oder einem Blutprodukt.

2. Verwendung gemäß Anspruch 1, worin das Medium einen Benetzbarkeitswert von 72 dyn/cm aufweist.

3. Verwendung gemäß Anspruch 1, worin die Fasern einen Durchmesser von 1 bis 8 µm haben.

4. Verwendung gemäß Anspruch 1, worin die Porosität des Mediums zwischen 75-95% beträgt.

5. Verwendung gemäß Anspruch 4, worin die Porosität zwischen 85-90% beträgt.

6. Verwendung gemäß Anspruch 1, worin das Medium Poren in dem schmelzgeblasenen Vlies aufweist und worin die Mehrzahl der Poren 2-30 µm groß sind.

7. Verwendung gemäß Anspruch 6, worin die Mehrzahl der Poren 5-15 µm groß sind.

8. Verwendung gemäß Anspruch 1, worin die Fasern um einen Faktor von zwischen 1-10 komprimiert und mechanisch bearbeitet werden.

9. Verwendung gemäß Anspruch 1, worin das Medium mit nassem Dampf sterilisiert wird.

10. Verwendung gemäß Anspruch 1, worin das Medium vor Gebrauch konditioniert wird.

11. Verwendung gemäß Anspruch 9, worin das Medium vor der Sterilisation konditioniert wird.

12. Leukoreduktionsfilter enthaltend ein Medium, das ein schmelzgeblasenes Vlies aus Cycloolefincopolymerfasern umfasst, die konditioniert sind mit einer Lösung, die Tween® 20 in einer Menge zwischen 0,01 und 0,5% enthält.

13. Verfahren zur Konditionierung eines ein schmelzgeblasenes Vlies aus Cycloolefincopolymerfasern umfassenden Mediums mit Tween® 20, umfassend die Schritte:
Durchleiten einer Konditionierungslösung durch das Medium oder Einweichen des Mediums in einer Konditionierungslösung; wobei die Konditionierungslösung 0,01 bis 0,5% Tween® 20 enthält; und
Spülen des konditionierten Mediums mit physiologischer Kochsalzlösung oder Wasser für eine Zeitdauer, die ausreicht, um restliches Tween® 20 im Wesentlichen vollständig zu entfernen.

14. Verfahren gemäß Anspruch 13, weiter umfassend Sterilisation des Mediums; worin der Durchleitungsschritt vor dem Sterilisationsschritt erfolgt.

15. Verfahren gemäß Anspruch 13, weiter umfassend Sterilisation des Mediums; worin der Durchleitungsschritt nach dem Sterilisationsschritt erfolgt.

## Revendications

1. Utilisation d'un médium hydrophobe inerte comprenant une toile de fusion-soufflage non tissée de fibres de copolymère de cyclooléfine dans laquelle les fibres sont conditionnées avec une solution contenant du Tween® 20 en une quantité comprise entre 0,01 et 0,5 % et en une quantité non toxique pour l'homme pour l'élimination sélective de leucocytes du sang ou d'un produit sanguin.

2. Utilisation selon la revendication 1 dans laquelle le médium a une valeur de mouillabilité de 72 dynes/cm.

3. Utilisation selon la revendication 1 dans laquelle les fibres ont un diamètre de 1 à 8 µm.

4. Utilisation selon la revendication 1 dans laquelle la porosité du médium est comprise entre 75 et 95 %.

5. Utilisation selon la revendication 4 dans laquelle la porosité est comprise entre 85 et 90 %.

6. Utilisation selon la revendication 1 dans laquelle le médium comprend des pores dans la toile de fusion-soufflage et dans laquelle la majorité des pores sont de 2-30 µm.

7. Utilisation selon la revendication 6 dans laquelle la majorité des pores sont de 5-15 µm.

8. Utilisation selon la revendication 1 dans laquelle les fibres sont comprimées et transformées mécaniquement avec un facteur compris entre 1 et 10.

9. Utilisation selon la revendication 1 dans laquelle le médium est stérilisé à la vapeur humide.

10. Utilisation selon la revendication 1 dans laquelle le médium est conditionné avant utilisation.

11. Utilisation selon la revendication 9 dans laquelle le médium est conditionné avant stérilisation.

12. Filtre de leucoréduction comprenant un médium comprenant une toile de fusion-soufflage non tissée de fibres de copolymère de cyclooléfine conditionnées avec une solution contenant du Tween® 20 en une quantité comprise entre 0,01 et 0,5%.

13. Procédé de conditionnement d'un médium comprenant une toile de fusion-soufflage non tissée de fibres de copolymère de cyclooléfine avec du Tween® 20 comprenant les étapes consistant à :
faire circuler une solution de conditionnement dans le médium ou tremper le médium dans une solution de conditionnement ; la solution de conditionnement contenant 0,01 à 0,5 % de Tween® 20 ; et
rincer le médium conditionné avec de la solution saline ou de l'eau pendant une durée suffisante pour enlever pratiquement tout le Tween® 20 résiduel.

14. Procédé selon la revendication 13 comprenant en outre la stérilisation du médium ; dans lequel l'étape de circulation a lieu avant l'étape de stérilisation.

15. Procédé selon la revendication 13 comprenant en outre la stérilisation du médium ; dans lequel l'étape de circulation a lieu après l'étape de stérilisation.
